# EUROPEAN PATENT SPECIFICATION

(11) **EP 3 332 699 B1**
(45) Date of publication and mention of the grant of the patent: **30.11.2022**
(21) Application number: 16844122.8
(22) Date of filing: 15.08.2016
(51) Int. Cl.: A61B 5/02, A61B 5/00, A61B 5/021, A61B 5/022, A61B 5/0245, A61B 5/0255, A61B 5/024

(54) **PULSE WAVE DETECTOR**
PULSWELLENDETEKTOR
DÉTECTEUR D'ONDE DE POULS

(30) Priority: 07.09.2015 JP 2015175965
(43) Date of publication of application: 13.06.2018
(73) Proprietor: Omron Healthcare Co., Ltd., Muko-shi, Kyoto 617-0002 (JP)
(72) Inventor: BRIGHAM Brian, Muko-shi Kyoto 617-0002 (JP); FUKUTSUKA Masayuki, Muko-shi Kyoto 617-0002 (JP); YAMASHITA Shingo, Muko-shi Kyoto 617-0002 (JP); KITAGAWA Tsuyoshi, Muko-shi Kyoto 617-0002 (JP); OGURA Toshihiko, Muko-shi Kyoto 617-0002 (JP); WAKAMIYA Masayuki, Muko-shi Kyoto 617-0002 (JP)
(74) Representative: Isarpatent
(86) International application number: PCT/JP2016/073849
(87) International publication number: WO 2017/043259

(56) References cited:
- WO-A1-2008/087870
- JP-A- H1 057 323
- JP-A- 2005 040 259
- JP-A- 2009 240 511

## Description

### Technical Field

The present invention relates to a pulse wave detecting device.

### Background Art

A biological information measuring device is known that, in a state where a pressure sensor is directly contacted with a living body portion through which an artery such as the radial artery in the wrist passes, can measure biological information such as the pulse and the blood pressure by using information detected by the sensor (for example, see JP-A-2008- 168054 and JP-UM-A-04-51907).

JP-A-2008-168054 discloses a biological information measuring device in which, in a state where the device is attached to the wrist, a portion of a housing that is to be wound around to the side of the back of the hand is formed into an S-like shape, whereby, while avoiding the ulna during the attachment of the device to the wrist, the attachment can be stably maintained.

JP-A-2008-168054 further discloses a configuration where the housing of the biological information measuring device is fixed to the wrist by an auxiliary belt in place of the formation of the portion of the housing that is to be wound around the hand back side, into an S-like shape.

JP-UM-A-04-51907 discloses a biological information measuring device that includes a housing in which only a pressure sensor portion is projected toward the forward end of the hand, and that is used while fixing the housing to the wrist by a belt.

Another biological information measuring device is described in WO 2008/087870 A1.

### Summary of the Invention

### Technical Problem

In the biological information measuring device which is disclosed in JP-A-2008-168054, and which is to be attached to the wrist by using the auxiliary belt, the housing in which the pressure sensor is mounted has an approximately uniform width in the peripheral direction of the wrist (hereinafter, referred to as the circumferential direction). In view of the accuracy of detection of a pressure pulse wave, it is preferable that the pressure sensor is as close as possible to the palmar crease existing in the base of the wrist.

In the configuration of JP-A-2008-168054 where the auxiliary belt is used, when the pressure sensor is as close as possible to the palmar crease, the housing covers the wrist root portion on the side of the back of the hand. Therefore, an operation of lifting the wrist to the side of the back of the hand is impeded by the housing, and, when the device is attached, the motion is limited.

JP-A-2008-168054 discloses also a configuration in which, in a state where the device is attached to the wrist, a housing portion that is wound around the back of the hand is formed into an S-like shape without using the auxiliary belt. Also in this configuration, the wrist root portion is covered by the housing in the right end of the side of the back of the hand, and therefore there is a possibility that an operation of lifting the wrist to the side of the back of the hand may be impeded by the housing.

When a highly stretchable housing is used as the housing which accommodates the pressure sensor, it is possible to prevent the motion of the wrist from being limited. In this configuration, however, there is a possibility that, after the device is fixed to the wrist, the positional displacement of the pressure sensor may occur because of extension or contraction of the housing. Therefore, it is difficult to accurately measure a pressure pulse wave.

The biological information measuring device of JP-UM-A-04-51907 has the configuration where the housing in which the pressure sensor is mounted exists only on the side of the palm of the hand. Therefore, the above described problems do not occur.

Although, in the above, the devices each of which detects a pressure pulse wave by using the pressure sensor have been described, similar problems occur also in a device which detects a volume pulse wave by, for example, using a photoelectric sensor.

The invention has been conducted in view of the above circumstances. It is an object of the invention to provide a pulse wave detecting device in which the motion of the hand in a state where the detector is attached to the wrist can be prevented from being impeded, and which can accurately detect a pulse wave.

### Solution to Problem

A pulse wave detecting device of the present invention, which is to be used while being attached to a wrist, includes: a housing which accommodates a pulse wave detection sensor that is configured to detect a pulse wave from a radial artery of a subject, and which is configured to be able to be wound along a circumferential direction of the wrist; and a band which is used for fixing the housing to the wrist, and which is lower in rigidity than the housing, the housing includes: a back-side portion which, in an attachment state where the housing is attached to the wrist by the band, is to be wound around the arm at a back side of a hand, a palm side portion which is to be wound around the arm at a palm side of the hand, an arm side end portion being an end portion of the housing which, in the attachment state, is on a side of an arm of the subject in a perpendicular direction perpendicular to the circumferential direction, and a hand side end portion being an end portion of the housing which, in the attachment state, is on that arm at a side of the hand of the subject in the perpendicular direction. According to the invention, in the attachment state, a position in the perpendicular direction of a part which is in the hand side end portion, and which constitutes the back-side portion, is closer to the arm of the subject than a position in the perpendicular direction of a part which is in a portion that constitutes the palm side portion, and which is closest to the side of the hand of the subject.

### Advantageous Effects of Invention

According to the invention, it is possible to provide a pulse wave detecting device in which the motion of the hand in a state where the detector is attached to the wrist can be prevented from being impeded, and which can accurately detect a pulse wave.

### Brief Description of Drawings

[Fig. 1] Fig. 1 is a diagram showing the external configuration of a biological information measuring device 100 for illustrating an embodiment of the invention.
[Fig. 2] Fig. 2 is a development view of a housing 20 of the biological information measuring device 100 shown in Fig. 1, taken along the circumferential direction X of the wrist.
[Fig. 3] Fig. 3 is a view illustrating a method of attaching the biological information measuring device 100 to the wrist.
[Fig. 4] Fig. 4 is a view illustrating the method of attaching the biological information measuring device 100 to the wrist.
[Fig. 5] Fig. 5 is a development view of a housing 20 of a biological information measuring device 100A which is a modification of the biological information measuring device 100 shown in Fig. 2, taken along the circumferential direction X of the wrist.
[Fig. 6] Fig. 6 is a development view of a housing 20 of a biological information measuring device 100B which is a modification of the biological information measuring device 100 shown in Fig. 2, taken along the circumferential direction X of the wrist.
[Fig. 7] Fig. 7 is a development view of a housing 20 of a biological information measuring device 100C which is a modification of the biological information measuring device 100.
[Fig. 8] Fig. 8 is a development view of a housing 20 of a biological information measuring device 100D which is a modification of the biological information measuring device 100.

### Description of Embodiments

Hereinafter, an embodiment of the invention will be described with reference to the drawings.

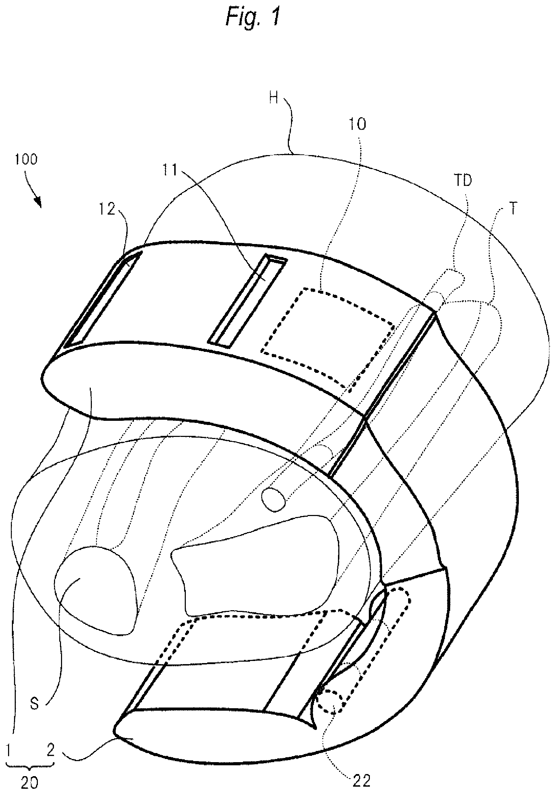

Fig. 1 is a diagram showing the external configuration of a biological information measuring device 100 for illustrating an embodiment of the invention. The biological information measuring device 100 is used while being attached to the wrist of the subject.

Fig. 1 shows the left wrist H of the subject. The near side of the figure coincides with the direction in which the hand of the subject exists. The upper side of the figure is in the direction along which the palm of the hand is oriented. In the wrist H, the radius T, the ulna S, and the radial artery TD are shown.

The biological information measuring device 100 includes a pulse wave detector 10 which detects a pulse wave (a pressure pulse wave or a volume pulse wave) from the radial artery TD that extends along the radius T of the wrist H of the subject, and measures biological information such as the blood pressure value and the pulse rate based on pulse waves detected by the pressure pulse wave detector 10.

The pulse wave detector 10 may have a known configuration. For example, the pulse wave detector 10 includes a pressure sensor and a mechanism which presses it against the skin, and detects a pressure pulse wave by using the pressure sensor. Alternatively, the pulse wave detector 10 includes a photoelectric sensor, and detects a volume pulse wave from a signal detected by the photoelectric sensor. The pressure sensor or the photoelectric sensor constitutes the pulse wave detection sensor.

The biological information measuring device 100 includes a housing 20 which accommodates: the pulse wave detector 10; and a biological information calculator that is not shown, and that calculates biological information such as the blood pressure value and the pulse rate based on the pulse wave detected by the pulse wave detector 10.

The biological information measuring device 100 is requested to have at least the pulse wave detector 10, and functions as the pulse wave detecting device. For example, the biological information calculator may be disposed in an apparatus other than the biological information measuring device 100.

The housing 20 is an approximately U-like housing which is configured so as to be woundable in the circumferential direction of the wrist H, and which is opened on the side of the ulna S of the wrist H. The housing 20 has a configuration in which the portion between the both ends in the circumferential direction of the wrist H does not cover the ulna S.

The housing 20 is configured by a housing 1 which accommodates the pulse wave detector 10, and a housing 2 which is coupled to the housing 1. The housing 2 is coupled to the housing 1 by fixation such as adhesion or welding, or detachably coupled to the housing 1 by coupling pins.

The housing 1 is configured mainly by a member having a first high rigidity in order to stabilize the position of the pulse wave detector 10 with respect to the radial artery TD in the state where the biological information measuring device 100 is attached to the wrist H, and to protect the pulse wave detector 10 including precision elements. As the member having the first rigidity, for example, a resin or a metal is used.

A part or whole of the housing 2 is configured by a member having a second rigidity which is lower than the first rigidity. For example, a portion of the housing 2 extending the outer circumferential surface (the surface opposite to the surface opposed to the wrist H) to a predetermined thickness is configured by the member having the second rigidity, and the other portion is configured by the member having the first rigidity.

As described above, a member which is lower in rigidity than the housing 1 is used in the outer circumferential surface of the housing 2, thereby causing the housing 2 to be easily deformed in accordance with the shape of the wrist H. As the member having the second rigidity, for example, an elastic member, a shape-memory alloy, or the like is used.

Although, in Fig. 1, the housing 20 which accommodates the pulse wave detector 10 is divided into the housing 1 and the housing 2, it may be formed as a single hosing configured by the same material.

A band fastener 22 for fixing a band 30 which will be described later, and which is used for securing the housing 20 to the wrist H is disposed on the inner circumferential surface (the surface opposed to the wrist H) of the housing 2. In the example of Fig. 1, the band fastener 22 is configured by a columnar metal fitting.

In the outer circumferential surface (the surface opposite to the surface opposed to the wrist H) of the housing 1, hole portions 11, 12 for engaging the band 30 with the housing 1 are disposed in juxtaposition with each other in the circumferential direction of the wrist H.

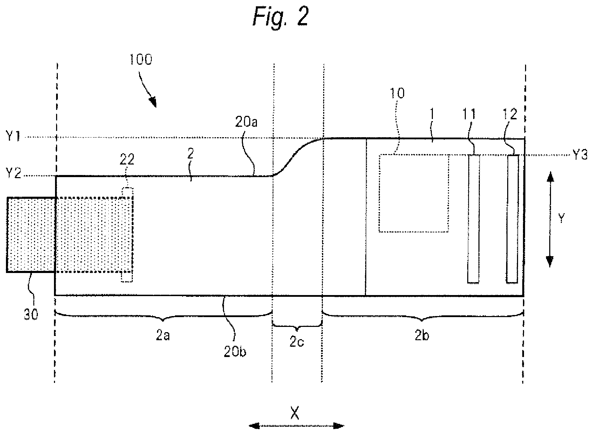

Fig. 2 is a development view of the housing 20 of the biological information measuring device 100 shown in Fig. 1, taken along the circumferential direction X of the wrist, and shows a view of the housing 20 as seen from the side of the outer circumferential surface. In Fig. 2, the direction perpendicular to the circumferential direction X is set as a direction Y.

In the attachment state where the biological information measuring device 100 is attached to the wrist, the arm of the subject is placed in the lower side in the direction Y in Fig. 2, and the hand of the subject is placed in the upper side in the direction Y.

As shown in Fig. 2, the band 30 is configured by a band member having a beltlike shape which elongates in the longitudinal direction (synonymous with the circumferential direction of the wrist H) of the housing 20, and which is lower in rigidity than the housing 20 (the rigidity is lower than the first rigidity and the second rigidity).

As the band member, for example, cloth or leather is used. Moreover, the band 30 is sufficiently smaller in thickness than the housing 20. The band 30 is more flexible in whole than the housing 20, and can be deformed more freely than the housing 20.

The basal end portion in the longitudinal direction of the band 30 is fixed to the housing 20. In a state where the band 30 is wound back from the basal end portion in the longitudinal direction toward the band fastener 22, specifically, the band is fixed to the band fastener 22 while portions of the same surface are bonded or sewn to each other. The tip end portion in the longitudinal direction of the band 30 is in a free state where the portion is not supported by any member.

The method of fixing the basal end portion of the band 30 of the housing 20 is not limited to the above described one. For example, a configuration may be employed where the basal end portion of the band 30 is fixed to the housing 20 by using screws or the like.

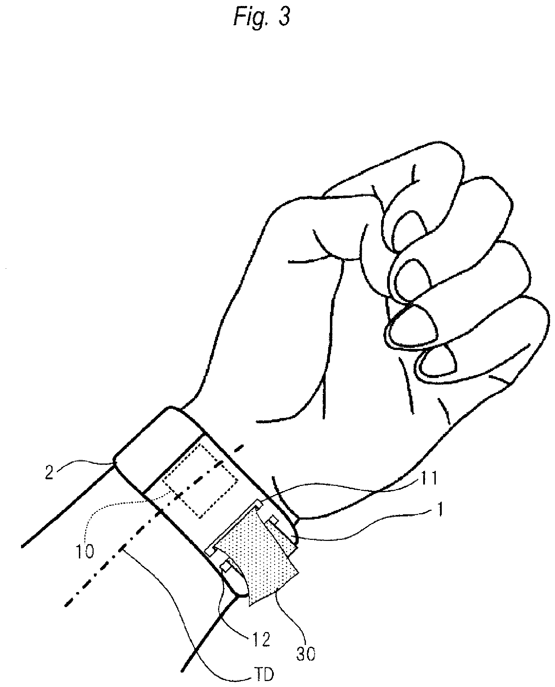

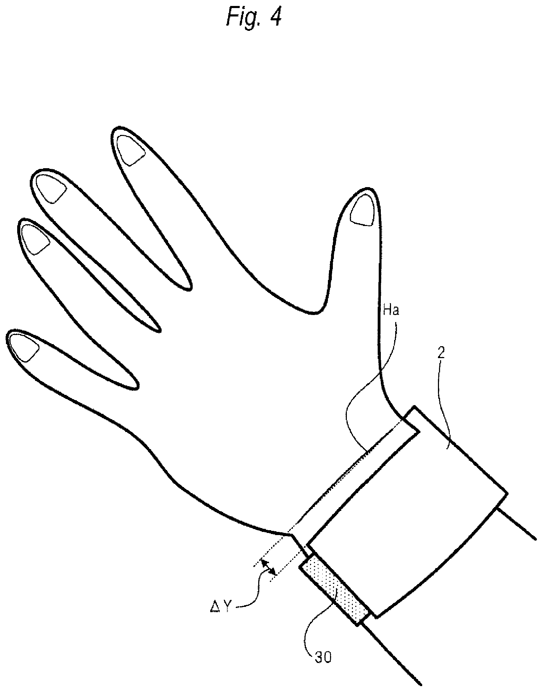

Figs. 3 and 4 are views illustrating the method of attaching the biological information measuring device 100 to the wrist. Fig. 3 is a view of the attachment state of the biological information measuring device 100 as seen from the side of the hand palm of the subject. Fig. 4 is a view of the attachment state of the biological information measuring device 100 as seen from the side of the back of the hand of the subject.

In a state where the housing 20 is provisionally placed on the wrist, as shown in Fig. 3, the subject causes the tip end of the band 30 to move around from the side of the back of the hand toward the palm of the hand, inserts the tip end into the hole portion 12, and pulls out the tip end of the band 30 from the hole portion 11. This state is shown in Figs. 3 and 4.

Although not illustrated, a hook portion in which fabrics are raised in hook-like shapes to constitute a hook and loop fastener, and a loop portion in which fabrics are densely raised in loop-like shapes to constitute the hook and loop fastener are formed on the surface opposite to the surface of the band 3 which is opposed to the wrist H.

In the state of Fig. 3, the subject strongly pulls the tip end portion of the band 30, and tightly winds the band 30 around the wrist H. Then, the subject causes the tip end portion of the band 30 to move around toward the back of the hand, and applies the loop portion disposed on the band 30 to the hook portion disposed on the band 30, thereby completing the attachment of the housing 20 to the wrist H by the band 30.

Returning to the description of Fig. 2, the housing 20 is configured by: a back-side portion 2a which is a portion to be wound around the hand back side of the wrist H of the subject in a state where the housing is attached to the wrist by the band 30; a palm side portion 2b which is a portion to be wound around the hand palm side of the wrist H of the subject in the state; and a coupling portion 2c which connects the back-side portion 2a and the palm side portion 2b together.

The back-side portion 2a means a portion of the housing 20 which overlaps with the hand of the subject in a plan view of an arbitrary plane in the case where the palm of the hand is made parallel to the plane in the state where the housing 20 is attached to the wrist H.

The palm side portion 2b means a portion of the housing 20 which overlaps with the hand of the subject in a plan view of an arbitrary plane in the case where the palm of the hand is made parallel to the plane in the state where the housing 20 is attached to the wrist.

An end portion of the housing 20 which is on the side of the hand of the subject in the direction Y in the state where the housing 20 is attached to the wrist H is called the hand side end portion, and denoted by a reference numeral 20a in Fig. 2. Moreover, an end portion of the housing 20 which is on the side of the arm of the subject in the direction Y in the state is called the arm side end portion, and denoted by a reference numeral 20b in Fig. 2.

The position Y2 in the direction Y of the part which is in the hand side end portion 20a, and which constitutes the back-side portion 2a is closer to the arm than the position Y1 in the direction Y of the part which is in the hand side end portion 20a, and which constitutes the palm side portion 2b.

The position Y3 in the direction Y of the end portion of the pulse wave detector 10 which is on the side of the hand of the subject is closer to the hand of the subject than the position Y2.

In the state where the thus configured housing 20 is attached to the wrist H, on the side of the back of the hand, as shown in Fig. 4, the hand side end portion 20a of the housing 20 is located at a position which is displaced toward the arm from the boundary Ha between the wrist and the hand by ΔY that is the difference between the position Y1 and the position Y2. Even when the subject performs an operation of lifting the hand to the side of the back of the hand, therefore, the operation is not impeded.

Moreover, the position Y3 in the direction Y of the end portion of the pulse wave detector 10 which is on the side of the hand is closer to the hand than the position Y2. Therefore, the pulse wave detector 10 can be sufficiently made close to the palmar crease, and the accuracy of detection of a pressure pulse wave can be improved.

According to the biological information measuring device 100, namely, the motion of the hand in the state where the device is attached to the wrist can be prevented from being impeded, and a pulse wave can be accurately detected.

In the housing 20, the position in the direction Y of the part which is in the arm side end portion 20b, and which constitutes the back-side portion 2a, and that in the direction Y of the part which constitutes the palm side portion 2b are identical with each other (the positions may not be strictly identical with each other, and may involve some tolerances).

Namely, the arm side end portion 20b has a linear shape which elongates in the circumferential direction X, and the position in the direction Y of the arm side end portion 20b is identical in the circumferential direction X (the position may involve some tolerances).

Even when the part which is in the arm side end portion 20b, and which constitutes the back-side portion 2a is displaced toward the hand of the subject with respect to, for example, the part which is in the arm side end portion 20b, and which constitutes the palm side portion 2b, it is possible to achieve the effect that the motion of the wrist is not impeded.

According to the configuration of Fig. 2, the position of the arm side end portion 20b is identical in the circumferential direction X, and therefore the width of the housing 20 in the direction Y can be increased as a whole. As a result, it is possible to achieve effects such as those that the durability of the housing 20 is improved, and that the attachment of the housing 20 can be stably performed.

In Fig. 2, the whole of the part which constitutes the palm side portion 2b of the hand side end portion 20a is closer to the side of the hand than the position Y2. As far as the part which constitutes the palm side portion 2b of the hand side end portion 20a is partially closer to the side of the hand of the subject than the position Y2, however, the motion of the wrist can be prevented from being impeded, while making the pulse wave detector 10 close to the palmar crease.

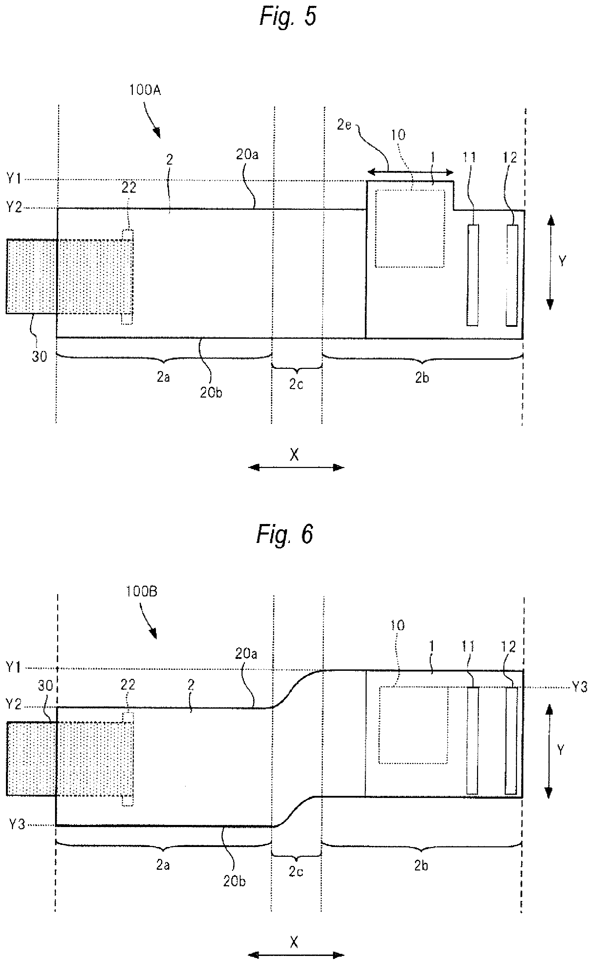

Fig. 5 is a development view of a housing 20 of a biological information measuring device 100A which is a modification of the biological information measuring device 100.

In the biological information measuring device 100A, in the part which constitutes the back-side portion 2a of the hand side end portion 20a, only the part (the part in the range of the reference numeral 2e) in which the pulse wave detector 10 is accommodated is closer to the side of the hand of the subject than the position Y2.

In the biological information measuring device 100A, as described above, the position Y2 in the direction Y of the part which is in the hand side end portion 20a, and which constitutes the back-side portion 2a is closer to the side of the arm of the subject than the position Y1 in the direction Y of the part (the part in the range of the reference numeral 2e in Fig. 5) which is closest to the side of the hand of the subject among parts constituting the palm side portion 2b.

Also in this configuration, it is possible to achieve an effect that, while making the pulse wave detector 10 close to the palmar crease in the range of the reference numeral 2e, the motion of the hand is prevented from being impeded by the distance difference between the position Y1 and the position Y2 on the side of the back of the hand.

According to the biological information measuring device 100A, moreover, the position of the arm side end portion 20b is identical in the circumferential direction X (the position may not be strictly identical, and may involve some tolerances). Similarly with the biological information measuring device 100, therefore, the width of the housing 20 in the direction Y can be ensured, and the attachability to the wrist can be improved.

Fig. 6 is a development view of a housing 20 of a biological information measuring device 100B which is a modification of the biological information measuring device 100.

The biological information measuring device 100B has a configuration where, in the biological information measuring device 100, the shape of the arm side end portion 20b is identical with that of the hand side end portion 20a.

Namely, the housing 20 has a configuration which elongates into an approximately S-like shape in the circumferential direction X, and the width in the direction Y is constant in the circumferential direction X (the width may involve some tolerances).

According to the biological information measuring device 100B, similarly with the biological information measuring device 100, it is possible to achieve an effect that, while making the pulse wave detector 10 close to the palmar crease, the motion of the hand is prevented from being impeded on the side of the back of the hand.

According to the biological information measuring device 100B, moreover, the width in the direction Y of the housing 20 is constant. Therefore, the rigidity of the housing 20 can be enhanced to improve the durability, and the attachability to the wrist can be improved. Furthermore, the design of the housing 20 can be enhanced.

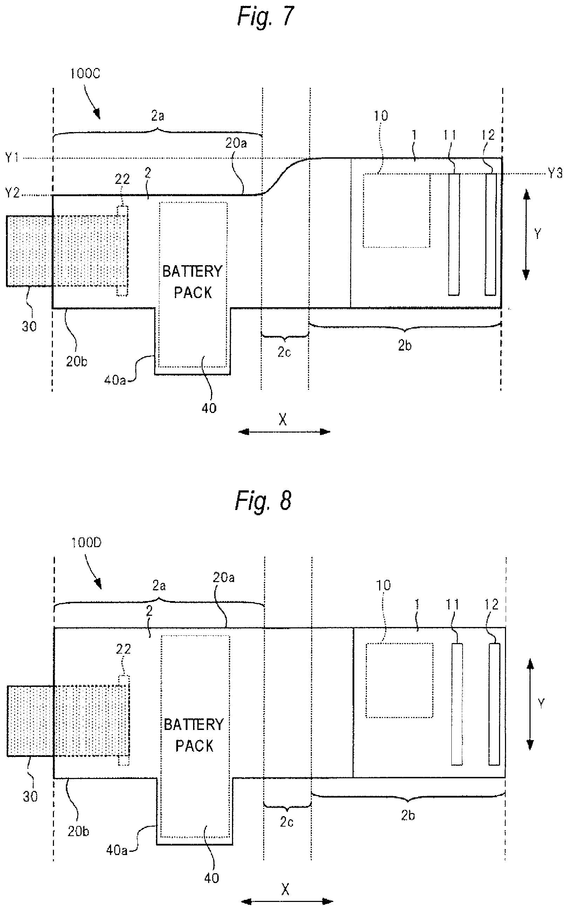

Fig. 7 is a development view of a housing 20 of a biological information measuring device 100C which is a modification of the biological information measuring device 100.

In the biological information measuring device 100C, the shape of the back-side portion 2a of the housing 20 is different from that in the biological information measuring device 100. Specifically, a part of the back-side portion 2a has a projecting part (convex part) 40a which projects in the direction Y. Namely, the width of the back-side portion 2a in the direction Y is not constant, and the width is partly increased by the projecting part 40a.

A battery pack 40 which is a battery for supplying power to the biological information measuring device 100C is accommodated in a housing portion in which the position in the circumferential direction X is identical with that of the projecting part 40a.

According to the thus configured biological information measuring device 100C, the projecting part 40a is formed in the back-side portion 2a of the housing 20, thereby enabling a portion of the arm of the subject in the vicinity of the wrist to be supported by the projecting part 40a. Therefore, the attachability of the housing 20 to the wrist can be improved.

Since the battery pack 40 is incorporated in the projecting part 40a, moreover, batteries having a large capacity can be used as the battery pack 40. As a result, the operating time of the biological information measuring device 100C can be prolonged, and this is effective particularly in the case where biological information is always measured.

Because the battery pack 40 is accommodated in the projecting part 40a, furthermore, also an effect that, in a work of replacement of the battery pack 40, the battery pack 40 can be easily taken out is achieved.

In the biological information measuring device 100C, it is preferable to form the surface of the portion of the housing 20 in which the battery pack 40 is accommodated, and which is to be in contact with the wrist of the subject, into a curved surface extending along the curve of the hand, in place of a flat surface. According to the configuration, a more stable support is enabled. Moreover, the feeling of attachment of the housing 20 can be improved.

The configuration of the back-side portion 2a shown in Fig. 7 may be applied also to the biological information measuring device 100A of Fig. 5, and the biological information measuring device 100B of Fig. 6.

The above-described effects which are achieved by the biological information measuring device 100C can be achieved also by a configuration where the hand side end portion 20a of the housing 20 is linear as shown in Fig. 8. In the biological information measuring device 100C, namely, the hand side end portion 20a of the housing 20 may have an arbitrary shape.

Also in this configuration, a portion of the arm of the subject in the vicinity of the wrist can be supported by the projecting part 40a, and the attachability of the housing 20 to the wrist can be improved. In the biological information measuring device 100D shown in Fig. 8, the battery pack 40 can be made larger than that of the biological information measuring device 100C.

The presently disclosed embodiment should be considered in all respects to be illustrative and not restrictive. The scope of the invention is indicated by the appended claims rather than the foregoing description, and all changes which come within the meaning and range of equivalents thereof are intended to be embraced therein.

As described above, the following matters are disclosed in the description.

The disclosed pulse wave detecting device is a pulse wave detecting device which is to be used while being attached to a wrist, and which includes: a housing which accommodates a pulse wave detection sensor that is configured to detect a pulse wave from a radial artery of a subject, and which is configured to be able to be wound along a circumferential direction of the wrist; and a band which is used for fixing the housing to the wrist, and which is lower in rigidity than the housing, the housing includes: a back-side portion which, in an attachment state where the housing is attached to the wrist by the band, is to be wound around a back side of a hand; and a palm side portion which is to be wound around a palm side of the hand, and, in the attachment state, a position in a direction of a part: which is in a hand side end portion that is an end portion of the housing, the end portion that is on a side of the hand of the subject in the direction; and which constitutes the back-side portion, is closer to an arm of the subject than a position in the direction of a part: which is in a portion that constitutes the palm side portion; and which is closest to the side of the hand of the subject, the direction that is perpendicular to the circumferential direction.

The disclosed pulse wave detecting device includes the configuration where a position of an arm side end portion which is an end portion of the housing that, in the attachment state, is on a side of the arm of the subject in the perpendicular direction is identical in the circumferential direction.

In the disclosed pulse wave detecting device, a width of the back-side portion in the perpendicular direction is equal to a width of the palm position in the perpendicular direction.

In the disclosed pulse wave detecting device, the back-side portion includes a projecting part which projects in the perpendicular direction on a side of the arm of the subject in the perpendicular direction, and a battery for power supply is accommodated in a portion of the housing which is at a same position as the projecting part in the circumferential direction.

The disclosed pulse wave detecting device is a pulse wave detecting device which is to be used while being attached to a wrist, and which includes a housing which accommodates a pulse wave detection sensor that is configured to detect a pulse wave from a radial artery of a subject, and which is configured to be able to be wound along a circumferential direction of the wrist, the housing includes: a back-side portion which, in an attachment state where the housing is attached to the wrist, is to be wound around a back side of a hand; and a palm side portion which is to be wound around a palm side of the hand, the back-side portion includes a projecting part which projects in the perpendicular direction on a side of the arm of the subject in the perpendicular direction, and a battery for power supply is accommodated in a portion of the housing which is at a same position as the projecting part in the circumferential direction.

### Industrial Applicability

According to the invention, it is possible to provide a pulse wave detecting device in which the motion of the hand in a state where the detector is attached to the wrist can be prevented from being impeded, and which can accurately detect a pulse wave.

Although the invention has been described with reference to the specific embodiment, the invention is not limited to the embodiment, and various changes can be made without departing from the technical spirit of the disclosed invention.

The application is based on Japanese Patent Application (No. 2015-175965) filed September 7, 2015.

### Reference Signs List

- 100, 100A, 100B: biological information measuring device
- 10: pulse wave detector
- 11, 12: hole portion
- 20: housing
- 22: band fastener
- T: radius
- S: ulna
- TD: radial artery
- 30: band
- 2a: back-side portion
- 2b: palm side portion
- 2c: coupling portion
- 20a: hand side end portion
- 20b: arm side end portion
- X: circumferential direction of wrist
- Y: direction perpendicular to circumferential direction

## Claims

1. A pulse wave detecting device (100; 100A; 100B) which is to be used while being attached to a wrist (H), the pulse wave detecting device (100; 100A; 100B) comprising:
a housing (20) which accommodates a pulse wave detection sensor (10) for detecting a pulse wave from a radial artery (TD) of a subject, and which is configured to be able to be wound along a circumferential direction (X) of the wrist (H); and
a band (30) for fixing the housing (20) to the wrist (H), and which is lower in rigidity than the housing (20), wherein
the housing (20) includes: a back-side portion (2a) which, in an attachment state where the housing (20) is attached to the wrist (H) by the band (30), is to be wound around the arm at a back side of a hand, a palm side portion (2b) which is to be wound around the arm at a palm side of the hand, an arm side end portion (20b) being an end portion of the housing (20) which, in the attachment state, is on a side of an arm of the subject in a perpendicular direction (Y) perpendicular to the circumferential direction (X), and a hand side end portion (20a) being an end portion of the housing (20) which, in the attachment state, is on an arm at a side of the hand of the subject in the perpendicular direction (Y),
**characterized in that**
in the attachment state, a position in the perpendicular direction (Y) of a part which is in the hand side end portion (20a), and which constitutes the back-side portion (2a), is closer to the arm of the subject than a position in the perpendicular direction (Y) of a part which is in a portion that constitutes the palm side portion (2b), and which is closest to the side of the hand of the subject.

2. The pulse wave detecting device according to claim 1, wherein
a position in the perpendicular direction of the arm side end portion (20b) is identical in the circumferential direction.

3. The pulse wave detecting device according to claim 1, wherein
the housing (20) elongates into a S-like shape in the circumferential direction.

4. The pulse wave detecting device according to claim 1 or 3, wherein
a width of the back-side portion (2a) in the perpendicular direction is equal to a width of the palm side portion (2b) in the perpendicular direction.

5. The pulse wave detecting device according to any one of claims 1 to 4, wherein
the back-side portion (2a) includes a projecting part (40a) which projects in the perpendicular direction on a side of the arm of the subject in the perpendicular direction, and
a battery (40) for power supply is accommodated in a portion of the housing (20) which is at a same position as the projecting part (40a) in the circumferential direction.

## Patentansprüche

1. Pulswellendetektionsvorrichtung (100; 100A; 100B), die zu verwenden ist, während sie an einem Handgelenk (H) angebracht ist, wobei die Pulswellendetektionsvorrichtung (100; 100A; 100B) umfasst:
ein Gehäuse (20), das einen Pulswellendetektionssensor (10) zum Detektieren einer Pulswelle von einer Speichenarterie (TD) eines Probanden aufnimmt und das so ausgeführt ist, dass es entlang einer Umfangsrichtung (X) des Handgelenks (H) herum gelegt werden kann; und
ein Band (30) zum Befestigen des Gehäuses (20) an dem Handgelenk (H), das eine geringere Steifigkeit als das Gehäuse (20) aufweist, wobei
das Gehäuse (20) umfasst: einen rückseitigen Abschnitt (2a), der in einem Anbringungszustand, in dem das Gehäuse (20) durch das Band (30) an dem Handgelenk (H) angebracht ist, auf einer Rückseite einer Hand um den Arm herum zu legen ist, einen handflächenseitigen Abschnitt (2b), der auf einer Handflächenseite der Hand um den Arm herum zu legen ist, einen armseitigen Endabschnitt (20b), der ein Endabschnitt des Gehäuses (20) ist, der sich in dem Anbringungszustand auf einer Seite eines Arms des Probanden in einer senkrechten Richtung (Y) senkrecht zur Umfangsrichtung (X) befindet, und einen handseitigen Endabschnitt (20a), der ein Endabschnitt des Gehäuses (20) ist, der sich in dem Anbringungszustand an einem Arm auf einer Seite der Hand des Probanden in der senkrechten Richtung (Y) befindet,
**dadurch gekennzeichnet, dass**
in dem Anbringungszustand eine Position in der senkrechten Richtung (Y) eines Teils, der sich in dem handseitigen Endabschnitt (20a) befindet und der den rückseitigen Abschnitt (2a) bildet, näher an dem Arm des Probanden liegt als eine Position in der senkrechten Richtung (Y) eines Teils, der sich in einem Abschnitt befindet, der den handflächenseitigen Abschnitt (2b) bildet, und der der Seite der Hand des Probanden am nächsten liegt.

2. Pulswellendetektionsvorrichtung nach Anspruch 1, wobei eine Position in der senkrechten Richtung des armseitigen Endabschnitts (20b) in der Umfangsrichtung identisch ist.

3. Pulswellendetektionsvorrichtung nach Anspruch 1, wobei sich das Gehäuse (20) in der Umfangsrichtung zu einer S-Form erstreckt.

4. Pulswellendetektionsvorrichtung nach Anspruch 1 oder 3, wobei
eine Breite des rückseitigen Abschnitts (2a) in der senkrechten Richtung gleich einer Breite des handflächenseitigen Abschnitts (2b) in der senkrechten Richtung ist.

5. Pulswellendetektionsvorrichtung nach einem der Ansprüche 1 bis 4, wobei
der rückseitige Abschnitt (2a) einen vorstehenden Teil (40a) aufweist, der in der senkrechten Richtung auf einer Seite des Arms des Probanden in der senkrechten Richtung vorsteht, und
eine Batterie (40) zur Stromversorgung in einem Abschnitt des Gehäuses (20) aufgenommen ist, der sich in der Umfangsrichtung an derselben Position wie der vorstehende Teil (40a) befindet.

## Revendications

1. Dispositif de détection d'onde de pouls (100 ; 100A ; 100B) qui doit être utilisé tout en étant fixé à un poignet (H), le dispositif de détection d'onde de pouls (100 ; 100A ; 100B) comprenant :
un boîtier (20) qui loge un capteur de détection d'onde de pouls (10) permettant de détecter une onde de pouls provenant d'une artère radiale (TD) d'un sujet, et qui est configuré pour pouvoir être enroulé dans une direction circonférentielle (X) du poignet (H) ; et
une bande (30) permettant de fixer le boîtier (20) au poignet (H) et dont la rigidité est inférieure à celle du boîtier (20), dans lequel :
le boîtier (20) comporte : une partie côté dos (2a) qui, dans un état de fixation où le boîtier (20) est fixé au poignet (H) par la bande (30), doit être enroulée autour du bras, du côté dos d'une main, une partie côté paume (2b) qui doit être enroulée autour du bras du côté paume de la main, une partie d'extrémité côté bras (20b) étant une partie d'extrémité du boîtier (20) qui, à l'état de fixation, se trouve du côté d'un bras du sujet dans une direction perpendiculaire (Y), perpendiculaire à la direction circonférentielle (X), et une partie d'extrémité côté main (20a) étant une partie d'extrémité du boîtier (20) qui, à l'état de fixation, se trouve sur un bras du côté de la main du sujet dans la direction perpendiculaire (Y),
**caractérisé en ce que** :
à l'état de fixation, une position dans la direction perpendiculaire (Y) d'une partie qui se trouve dans la partie d'extrémité côté main (20a) et qui constitue la partie côté dos (2a) est plus proche du bras du sujet qu'une position dans la direction perpendiculaire (Y) d'une partie qui est une partie qui constitue la partie côté paume (2b) et qui est la plus proche du côté de la main du sujet.

2. Dispositif de détection d'onde de pouls, selon la revendication 1, dans lequel :
une position dans la direction perpendiculaire de la partie d'extrémité côté bras (20b) est identique dans la direction circonférentielle.

3. Dispositif de détection d'onde de pouls, selon la revendication 1, dans lequel :
le boîtier (20) s'étend en une forme de S dans la direction circonférentielle.

4. Dispositif de détection d'onde de pouls, selon la revendication 1 ou 3, dans lequel :
une largeur de la partie côté dos (2a) dans la direction perpendiculaire est égale à une largeur de la partie côté paume (2b) dans la direction perpendiculaire.

5. Dispositif de détection d'onde de pouls, selon l'une quelconque des revendications 1 à 4, dans lequel :
la partie côté dos (2a) comporte une partie saillante (40a) qui fait saillie dans la direction perpendiculaire d'un côté du bras du sujet dans la direction perpendiculaire, et
une batterie (40) d'alimentation électrique est logée dans une partie du boîtier (20) qui se trouve à la même position que la partie saillante (40a) dans la direction circonférentielle.
